# FASCICULE DE BREVET EUROPEEN

(11) **EP 4 659 669 B1**
(45) Date de publication et mention de la délivrance du brevet: **23.09.2026**
(21) Numéro de dépôt: 25178916.0
(22) Date de dépôt: 27.05.2025
(51) Int. Cl.: A61B 5/145, A61B 5/1477, A61B 5/00

(54) **DISPOSITIF DE SURVEILLANCE DE LA SUDATION D'UN ÊTRE VIVANT**
VORRICHTUNG ZUR ÜBERWACHUNG DER ENTSTEHUNG EINES LEBEWESENS
DEVICE FOR MONITORING THE PERSPIRATION OF A LIVING BEING

(30) Priorité: 04.06.2024 FR 2405840
(43) Date de publication de la demande: 10.12.2025
(73) Titulaire: Commissariat à l'Energie Atomique et aux Energies Alternatives, 75015 Paris (FR)
(72) Inventeur: REVOL-CAVALIER, Frédéric, 38054 GRENOBLE CEDEX 09 (FR)
(74) Mandataire: INNOV-GROUP

(56) Documents cités:
- US-A1- 2015 112 165
- VAIBHAV JAIN ET AL: "A mass-customizable dermal patch with discrete colorimetric indicators for personalized sweat rate quantification", MICROSYSTEMS & NANOENGINEERING, vol. 5, no. 1, 17 June 2019 (2019-06-17), XP055643195, DOI: 10.1038/s41378-019-0067-0

## Description

### Domaine technique de l'invention

La présente invention se rapporte à un dispositif de surveillance de la sudation d'un être vivant.

### Etat de la technique

Différents capteurs de mesure sont désormais connus pour suivre en temps réel certains paramètres physiologiques d'un être vivant, comme la glycémie, la fréquence cardiaque... Récemment, des capteurs de sudation ont également été développés pour suivre les pertes hydriques subies par un être vivant. Ces capteurs peuvent permettre d'alerter sur une possible déshydratation, par exemple lors d'une activité physique, ou être utilisés à d'autres fins, par exemple pour mesurer le niveau de stress de l'être vivant. La demande de brevet US2022/257131 A1 décrit notamment la réalisation d'un dispositif portable, sous la forme d'un patch à appliquer contre la peau de l'être vivant. Le patch peut être équipé d'un système de mesure capable de mesurer des paramètres physiologiques de l'être vivant, par analyse des fluides corporelles, c'est-à-dire à partir de la sueur sécrétée par l'être vivant. Les paramètres surveillés sont par exemple le niveau en sodium, les lactates, le taux de glucose...

Le brevet US10405794B2 décrit un détecteur de sudation utilisant notamment un capillaire en serpentin capable de se remplir du fluide corporel, des électrodes étant réparties le long du capillaire pour déterminer le volume de fluide capturé et suivre le volume de sueur sécrété.

Les solutions antérieures ne permettent souvent pas un suivi précis du volume de sueur généré par l'être vivant. Dans le document US10405794B2**,** une fois qu'un premier palier de volume est atteint, la solution proposée ne permet pas de pouvoir suivre le remplissage du capillaire au cours du temps.

La demande de brevet US2015/112165A1 décrit un dispositif capable de mesurer un taux de sudation.

La publication référencée ci-dessous décrit la réalisation d'un patch permettant un suivi en temps réel de la sudation. Ce patch présente la particularité de disposer de plusieurs branches de tailles différentes, capables chacune de changer de couleur lorsqu'elles arrivent à saturation.

Jain, V., Ochoa, M., Jiang, H. et al. A mass-customizable dermal patch with discrete colorimetric indicators for personalized sweat rate quantification. Microsyst Nanoeng, 29 (2019). https://doi.org/10.1038/s41378-019-0067-0

Le but de l'invention est de proposer un dispositif de surveillance de la sudation d'un être vivant, permettant notamment de suivre le volume de sueur sécrété de manière plus précise que dans l'état de la technique.

### Exposé de l'invention

Ce but est atteint par un dispositif de surveillance de la sudation d'un être vivant, ladite sudation provoquant la création d'un fluide corporel, ledit dispositif comprenant un support présentant une surface active destinée à être appliquée contre la peau d'un être vivant, ledit dispositif comportant une unité de traitement et n capteurs, avec n supérieur ou égal à 2, chaque capteur étant agencé sur ladite surface active et relié à ladite unité de traitement pour fournir des données de mesure électrique, chaque capteur comportant un élément d'absorption apte à absorber un volume de fluide corporel, les capteurs étant choisis avec des capacités d'absorption en fluide corporel distinctes de sorte qu'un premier capteur arrivera à saturation en fluide corporel plus rapidement qu'un deuxième capteur, l'unité de traitement étant configurée pour déterminer une concentration ionique du fluide corporel, à partir de la valeur de la donnée de mesure électrique fournie par le premier capteur à saturation et du volume maximal de fluide corporel que l'élément d'absorption du premier capteur peut absorber.

Selon l'invention, l'unité de traitement est configurée pour suivre le remplissage en volume du deuxième capteur au cours du temps en tenant compte des données de mesure électrique fournies par ce deuxième capteur au cours du temps et de la concentration ionique déterminée à l'aide du premier capteur.

Selon une autre particularité, l'unité de traitement est configurée pour déterminer le volume de fluide corporel absorbé par le premier capteur en tenant compte de la concentration ionique du fluide corporel déterminée et des données de mesure électrique fournies par le premier capteur au cours du temps.

Selon une autre particularité, les données de mesure électrique sont des données d'impédance électrique et/ou de conductivité électrique du fluide corporel.

Selon une autre particularité, les capacités d'absorption distinctes des capteurs sont réalisées en jouant sur le type de matériau absorbant employé et/ou sur le volume de matériau absorbant employé et/ou sur la densité du matériau absorbant employé.

L'invention concerne également un procédé de surveillance de la sudation d'un être vivant, ladite sudation provoquant la création d'un fluide corporel, ledit procédé étant mis en œuvre avec un dispositif de surveillance tel que défini ci-dessus, le procédé consistant à déterminer la concentration ionique du fluide corporel, à partir de la valeur de la donnée de mesure électrique fournie par le premier capteur à saturation et du volume maximal de fluide corporel que l'élément d'absorption du premier capteur peut absorber.

Selon l'invention, le procédé comporte une étape de surveillance du remplissage en volume du deuxième capteur au cours du temps en tenant compte des données de mesure électrique fournies par ce deuxième capteur au cours du temps et de la concentration ionique déterminée à l'aide du premier capteur.

Selon une autre particularité, le procédé comporte une étape de détermination du volume de fluide corporel absorbé par le premier capteur en tenant compte de la concentration ionique du fluide corporel déterminée et des données de mesure électrique fournies par le premier capteur au cours du temps.

Selon une autre particularité, les données de mesure électrique sont des données d'impédance électrique et/ou de conductivité électrique du fluide corporel.

Selon une autre particularité, les capacités d'absorption distinctes des capteurs sont réalisées en jouant sur le type de matériau absorbant employé et/ou sur le volume de matériau absorbant employé et/ou sur la densité du matériau absorbant employé.

### Brève description des figures

D'autres caractéristiques et avantages vont apparaître dans la description détaillée qui suit faite en regard des dessins annexés dans lesquels :
- Les figures 1A et 1B représentent de manière schématique le dispositif de surveillance de la sudation conforme à l'invention ;
- La figure 2 illustre par un schéma le principe de fonctionnement du dispositif de surveillance de l'invention, selon une réalisation avantageuse ;
- La figure 3 représente un diagramme montrant les courbes de variation de l'impédance en fonction du volume de fluide absorbé pour des capteurs ayant des capacités d'absorption distinctes ;
- La figure 4 représente un diagramme montrant une courbe de variation de l'impédance à saturation mesurée par un capteur employé dans l'invention en fonction de la concentration ionique ;
- La figure 5 montre un diagramme illustrant la relation entre le volume absorbé par un élément d'absorption en fonction de la concentration ionique du fluide ;

### Description détaillée d'au moins un mode de réalisation

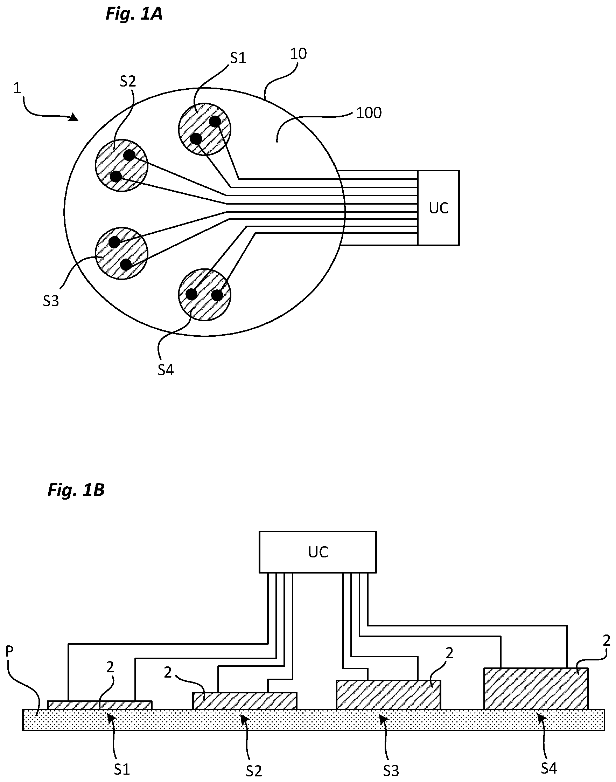

En référence à la figure 1A et à la figure 1B, l'invention vise un dispositif 1 de surveillance de la sudation d'un être vivant, par exemple un être humain. Par la sudation, l'être vivant dégage à travers la peau P un fluide corporel appelé sueur.

Le dispositif peut se présenter sous la forme d'un patch à appliquer sur la peau P de l'être vivant.

Le dispositif peut ainsi comporter un unique support 10 venant se coller sur la peau de l'être vivant. De manière non limitative, le support 10 peut avoir une surface comprise entre 5cm² et 15cm².

Le support 10 se présente par exemple sous la forme d'une bande ou disque de matière souple, présentant une surface dite active 100 destinée à être appliquée contre la peau de l'être vivant.

Selon l'invention, le dispositif comporte plusieurs capteurs S1, S2, S3, S4 (trois ou quatre capteurs sur les figures annexées), ces capteurs étant répartis sur ladite surface active 100 du support 10.

Le dispositif 1 comporte également une unité de traitement UC.

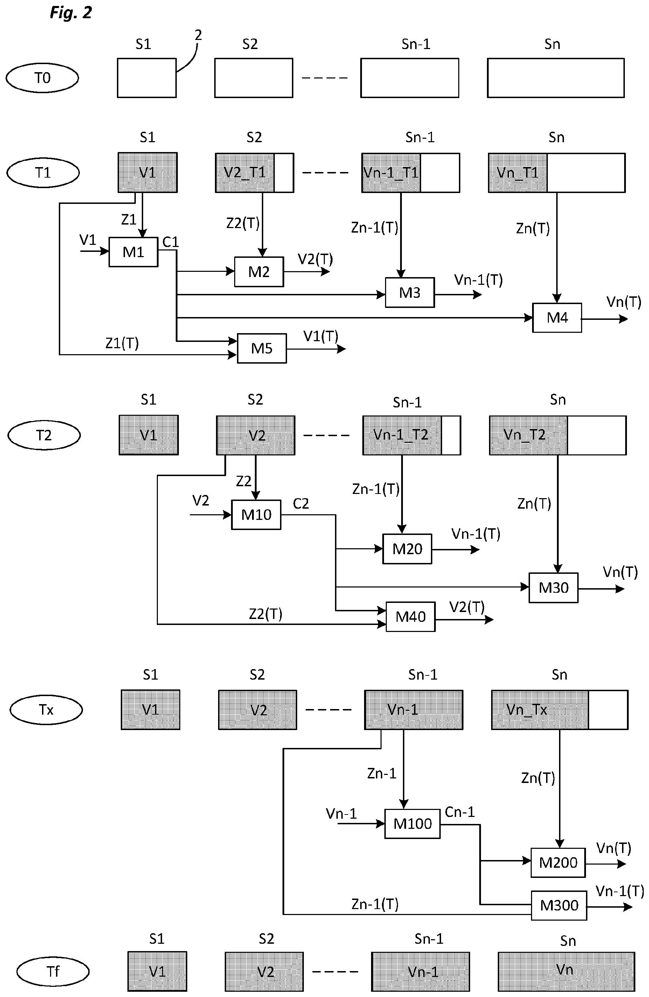

L'unité de traitement UC peut être intégrée au support 10, montée sur le support 10 ou déportée par rapport au support 10. Des moyens de communication filaire ou sans-fil peuvent être intégrés au support 10 pour envoyer les données mesurées par chaque capteur vers l'unité de traitement UC (lignes de connexion sur les figures 1A et 1B). Sur la figure 2, la représentation est schématique et permet d'illustrer le principe de réalisation de l'invention. Une connexion filaire est par exemple employée entre chaque capteur et l'unité de traitement UC.

L'unité de traitement UC peut être par exemple celle d'un objet portable intelligent, tel que par exemple un téléphone mobile, un ordinateur ou une montre intelligente.

Chaque capteur comporte un élément d'absorption 2 réalisé dans au moins un matériau absorbant. Cet élément est chargé d'absorber le fluide corporel émis par l'être vivant lorsque le dispositif est positionné, par sa surface active, contre la peau de l'être vivant. L'élément d'absorption 2 présente une capacité de saturation déterminée, c'est-à-dire qu'il est capable d'absorber un volume maximal de fluide corporel.

Selon un aspect particulier de l'invention, chaque capteur est utilisé pour mesurer un paramètre électrique au sein de son élément d'absorption. Ce paramètre électrique est par exemple l'impédance électrique (désignée Z) et/ou la conductivité électrique du fluide et/ou tout autre paramètre électrique pertinent. Dans la suite de la description et sur les dessins, on utilisera la mesure d'impédance électrique Z.

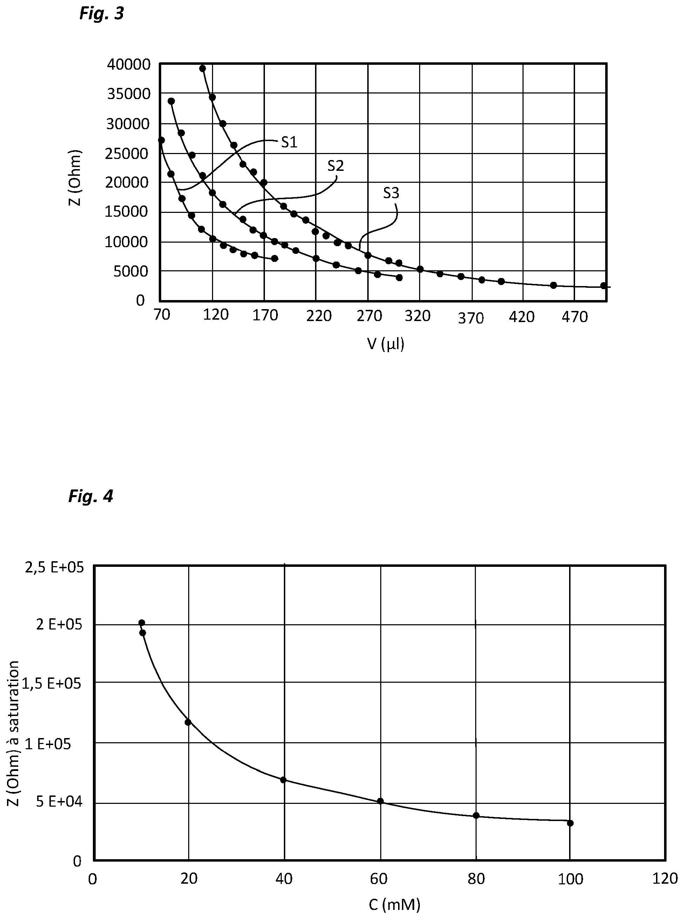

En liaison avec la figure 3, on constate en effet que l'impédance Z diminue lorsque le volume absorbé par l'élément d'absorption d'un capteur augmente. Autrement dit, plus l'élément d'absorption 2 absorbe de fluide corporel, plus l'impédance électrique mesurée par le capteur va diminuer, jusqu'à atteindre un palier bas, synonyme que l'élément d'absorption 2 est saturé en fluide. Sur cette figure 3, le capteur S1 a une capacité d'absorption plus faible que celle du capteur S2, celle-ci étant plus faible que celle du capteur S3. On constate alors que plus la capacité de saturation de l'élément d'absorption est importante (moins il sature vite en fluide), plus il atteint son palier d'impédance tardivement.

Le diagramme de la figure 4 montre en outre que l'impédance Z mesurée par un capteur à saturation diminue lorsque la concentration ionique (ici la concentration en NaCl) du fluide capturé augmente. Autrement dit, en plus du volume de fluide corporel capturé, la concentration ionique du fluide capturé a une incidence sur l'impédance Z mesurée. Il en ressort qu'un élément d'absorption 2 faiblement imbibé par un fluide ayant une concentration ionique élevée peut renvoyer un même signal électrique qu'un élément d'absorption 2 imbibé plus fortement par un fluide de plus faible concentration ionique. La mesure électrique de la conductivité du matériau (via la mesure d'impédance ou autre) est donc corrélée à la fois à la quantité de fluide présente dans le l'élément d'absorption 2 ainsi qu'à sa concentration.

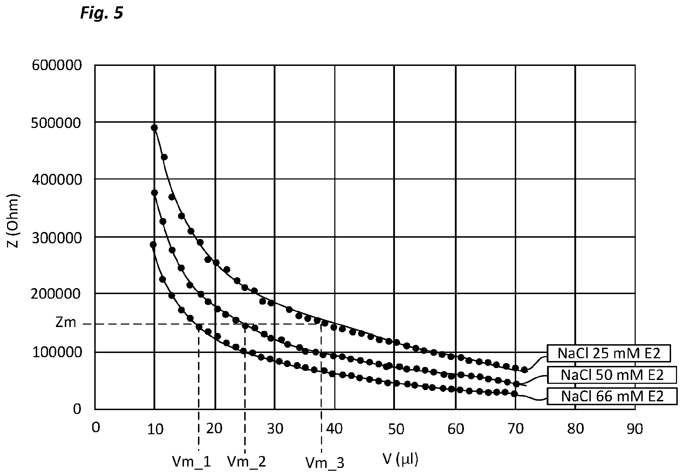

Partant de là, l'une des particularités du dispositif de l'invention est d'employer un premier capteur S1 disposant d'un élément d'absorption à faible capacité de saturation. Ainsi, comme ce capteur S1 sature rapidement en fluide, l'unité de traitement UC peut déduire immédiatement une concentration ionique du fluide capturé. Une fois la concentration déterminée, il est possible de remonter au volume présent dans l'élément d'absorption à chaque point de mesure à partir du diagramme de la figure 5. La figure 5 montre ainsi trois courbes, chacune pour une concentration ionique distincte, chaque courbe reliant l'impédance mesurée au volume de fluide absorbé. Sur cette figure, on constate notamment que trois volumes Vm_1, Vm_2, Vm_3 de fluide distinct, à trois niveaux de concentration ioniques distincts, permettent de mesurer une même impédance Zm.

L'ensemble des capteurs du dispositif est également choisi de manière à créer une gamme de saturation. Par gamme de saturation, on entend que les capteurs présentent tous une capacité d'absorption distincte et une zone de recouvrement suffisante d'un capteur à l'autre. Par zone de recouvrement, on entend que tous les capteurs commencent à se remplir en même temps, dès le début de l'utilisation du dispositif. D'un capteur à l'autre, les capacités d'absorption distinctes peuvent être créées en jouant sur le type de matériau absorbant et/ou sur le volume de matériau absorbant et/ou sur la densité de matériau absorbant.

Sur la figure 1B, de manière non limitative, les capteurs S1-S4 sont choisis pour se différencier entre eux par l'épaisseur de matériau absorbant utilisé pour leur élément d'absorption 2.

L'utilisation de plusieurs capteurs à capacités d'absorption distinctes permet notamment d'étendre la gamme de mesure :
- L'élément d'absorption du premier capteur S1 (avec la plus faible capacité d'absorption) va permettre une mesure quasi immédiate de la présence de fluide et permettre de déterminer le débit sudoral au départ de l'activité physique, dès l'apparition des premières gouttes de sueur.
- Le capteur suivant S2 (avec une capacité d'absorption plus importante que le premier), mettra plus de temps avant de commencer à afficher des valeurs de débit sudoral mais comme il se saturera plus tard, il va permettre d'augmenter la gamme de mesure en continuant à donner des mesures alors que le premier capteur sera saturé.
- Ce principe est dupliqué pour les autres capteurs du dispositif qui suivent dans la série (jusqu'à n capteurs). On va ainsi mesurer les quantités de sueur collectées en fonction de la capacité de collecte. Ce principe de multi capteurs de mesure permet d'augmenter la gamme de mesure.

Un autre avantage de l'utilisation de plusieurs capteurs est la redondance des mesures. En effet, si la capacité d'absorption des capteurs est différente d'un capteur a l'autre, des zones de recouvrement existent, ce qui permet d'avoir des mesures sur plusieurs capteurs en même temps. Cette redondance des mesures permet de s'assurer que les mesures effectuées sont fiables.

Un autre avantage lié à l'utilisation de plusieurs capteurs disposant de capacités d'absorption distinctes est qu'à chaque fois qu'un capteur se sature, il est possible de remonter à la concentration de la sueur moyenne collectée dans le capteur. Il est donc possible d'avoir un suivi régulier de la variation de la concentration de la sueur dans le temps.

On peut utiliser cette valeur de concentration mesurée pour affiner la quantité (le volume) de sueur collectée en fonction de sa concentration.

L'invention est décrite plus précisément en liaison avec la figure 2.

Sur la figure 2, le dispositif 1 comporte un nombre n de capteurs, avec n supérieur ou égal à 2. Les capteurs sont définis par leur élément d'absorption 2 de capacités distinctes.

Il faut noter que plus le dispositif 1 comporte un grand nombre de capteurs, plus l'échantillonnage sera important et donc plus la surveillance de la sudation sera précise.

Pour les n capteurs, on a par exemple :
- Le premier capteur S1 qui dispose d'une première capacité d'absorption, correspondant à un volume V1 maximal de fluide qu'il peut absorber.
- Le deuxième capteur S2 qui dispose d'une deuxième capacité d'absorption, distincte de la première capacité d'absorption, correspondant donc à un volume V2 choisi par exemple supérieur à V1 et qui correspond au volume maximal de fluide que ce deuxième capteur S2 peut absorber.
- Le capteur Sn qui dispose d'une énième capacité d'absorption, distincte de la capacité d'absorption du capteur Sn-1, correspondant donc à un volume Vn choisi par exemple supérieur à Vn-1 et qui correspond au volume maximal de fluide que ce le capteur Sn est capable d'absorber.

Pendant tout le processus, l'unité de traitement UC mesure l'impédance Z (ou autre paramètre électrique pertinent) au sein de l'élément d'absorption 2. Comme montré par la figure 3 et décrit ci-dessus, lorsque l'impédance atteint un palier bas, cela signifie que le capteur arrive à saturation.

Partant de là, le principe de fonctionnement est le suivant (sur la figure 2, la zone grisée correspond au niveau de remplissage de chaque capteur S1-Sn) :
A T0 : Chaque élément d'absorption 2 des capteurs S1-Sn est vide et n'a encore absorbé aucun fluide.
Entre T0 et T1 : L'activité physique a débuté et chaque élément d'absorption 2 des capteurs se remplit progressivement de fluide corporel.
A T1 : L'élément d'absorption du capteur S1 arrive à saturation le premier et son volume V1 correspondant à sa capacité d'absorption est plein. Comme le capteur S1 est saturé, l'unité de traitement UC connaît donc le volume V1 de sueur absorbé par le capteur S1. A saturation, l'unité de traitement UC détermine, à l'aide d'un module de calcul M1, la concentration ionique C1 du fluide capturé à partir de l'impédance Z1 mesurée et du volume V1.

La concentration C1 déterminée peut être employée par l'unité de traitement UC pour suivre le remplissage en volume V2(T) du deuxième capteur S2 au cours du temps et le remplissage en volume de chaque autre capteur du dispositif (Vn-1(T), Vn(T)), en tenant compte des impédances mesurées au niveau de chaque capteur au cours du temps (Z2(T), Zn-1(T), Zn(T)). Les modules de calcul M2, M3 et M4 sont exécutés par l'unité de traitement UC pour effectuer ces calculs.

En outre, la concentration ionique C1 déterminée peut être employée de manière rétroactive pour connaître la cinétique de remplissage en volume (V1(T)) du premier capteur S1 à l'aide de chaque point de mesure d'impédance acquis au cours du temps (Z1(T)). Un module de calcul M5 est exécuté par l'unité de traitement pour effectuer ce calcul. La figure 5 déjà décrite ci-dessus explique la relation entre ces différentes données.

A T2 : L'élément d'absorption du deuxième capteur S2 sature à son tour, par le volume V2. L'unité de traitement UC peut alors à nouveau déterminer, via un module de calcul M10, la concentration ionique C2 du fluide capturé par le capteur S2, en tenant compte de l'impédance mesurée Z2 à saturation.

De même, la concentration ionique C2 est employée par l'unité de traitement UC pour suivre le remplissage en volume (Vn-1(T) et Vn(T)) des autres capteurs non encore saturés, S3 à Sn, à l'aide des mesures d'impédance au cours du temps (Zn-1(T) et Zn(T)). Les modules de calcul M20 et M30 sont exécutés par l'unité de traitement UC pour effectuer ces calculs.

De même qu'à T1, il est également possible de remonter aux volumes V2(T) de fluide corporel absorbé au cours du temps par le deuxième capteur S2 à partir des points de mesure d'impédance Z2(T) et de la concentration ionique C2 déterminée. Le module de calcul M40 est exécuté par l'unité de traitement UC pour effectuer cette opération.

A Tx : Le capteur Sn-1 arrive à saturation. Le principe décrit ci-dessus est reproduit pour déterminer, à l'aide d'un module de calcul M100, la concentration ionique Cn-1 du fluide à l'aide du volume du capteur Sn-1 et de l'impédance Zn-1 mesurée à saturation du capteur Sn-1. L'unité de traitement est également configurée pour suivre le volume de remplissage du capteur Sn au cours du temps (Vn(T)), via le module de calcul M200 recevant en entrée la concentration ionique Cn-1 et l'impédance Zn(T) mesurée au cours du temps au niveau du capteur Sn.

De même qu'à T1 et T2, il est également possible de remonter aux volumes Vn-1(T) de fluide corporel absorbé au cours du temps par le capteur Sn-1 à partir des points de mesure d'impédance Zn-1(T) et de la concentration ionique Cn-1 déterminée. Le module de calcul M300 est exécuté par l'unité de traitement pour effectuer cette opération.

A Tf : Tous les capteurs S1-Sn sont remplis par le fluide corporel et arrivent donc à saturation.

Le principe de l'invention présente ainsi de nombreux avantages, parmi lesquels :
- Une solution permettant un suivi fiable de la sudation, dès les premiers instants de l'activité physique ;
- Une solution fiable et simple à mettre en place ;
- Une solution permettant un suivi de la sudation sur une large plage de mesures ;

## Revendications

1. Dispositif (1) de surveillance de la sudation d'un être vivant, ladite sudation provoquant la création d'un fluide corporel, ledit dispositif comprenant un support (10) présentant une surface active (100) destinée à être appliquée contre la peau (P) d'un être vivant, ledit dispositif comportant une unité de traitement (UC) et comportant également n capteurs, avec n supérieur ou égal à 2, chaque capteur (S1-Sn) étant agencé sur ladite surface active et relié à ladite unité de traitement (UC) pour fournir des données de mesure électrique, chaque capteur comportant un élément d'absorption (2) apte à absorber un volume de fluide corporel, les capteurs étant choisis avec des capacités d'absorption en fluide corporel distinctes de sorte qu'un premier capteur arrivera à saturation en fluide corporel plus rapidement qu'un deuxième capteur, l'unité de traitement (UC) étant configurée pour déterminer une concentration ionique du fluide corporel, à partir de la valeur de la donnée de mesure électrique fournie par le premier capteur (S1) à saturation et du volume maximal de fluide corporel que l'élément d'absorption (2) du premier capteur (S1) peut absorber, l'unité de traitement (UC) étant configurée pour suivre le remplissage en volume du deuxième capteur (S2) au cours du temps en tenant compte des données de mesure électrique fournies par ce deuxième capteur (S2) au cours du temps et de la concentration ionique déterminée à l'aide du premier capteur (S1).

2. Dispositif selon la revendication 1, **caractérisé en ce que** l'unité de traitement (UC) est configurée pour déterminer le volume de fluide corporel absorbé par le premier capteur (S1) en tenant compte de la concentration ionique du fluide corporel déterminée et des données de mesure électrique fournies par le premier capteur (S1) au cours du temps.

3. Dispositif selon la revendication 1 ou 2, **caractérisé en ce que** les données de mesure électrique sont des données d'impédance électrique et/ou de conductivité électrique du fluide corporel.

4. Dispositif selon l'une des revendications 1 à 3, **caractérisé en ce que** les capacités d'absorption distinctes des capteurs sont réalisées en jouant sur le type de matériau absorbant employé et/ou sur le volume de matériau absorbant employé et/ou sur la densité du matériau absorbant employé.

5. Procédé de surveillance de la sudation d'un être vivant, ladite sudation provoquant la création d'un fluide corporel, ledit procédé étant **caractérisé en ce qu'**il est mis en œuvre avec un dispositif (2) de surveillance tel que défini dans l'une des revendications précédentes, et **en ce qu'**il consiste à déterminer la concentration ionique du fluide corporel, à partir de la valeur de la donnée de mesure électrique fournie par le premier capteur (S1) à saturation et du volume maximal de fluide corporel que l'élément d'absorption du premier capteur peut absorber, ledit procédé comportant une étape de surveillance du remplissage en volume du deuxième capteur (S2) au cours du temps en tenant compte des données de mesure électrique fournies par ce deuxième capteur (S2) au cours du temps et de la concentration ionique déterminée à l'aide du premier capteur.

6. Procédé selon la revendication 5, **caractérisé en ce qu'**il comporte une étape de détermination du volume de fluide corporel absorbé par le premier capteur (S1) en tenant compte de la concentration ionique du fluide corporel déterminée et des données de mesure électrique fournies par le premier capteur (S1) au cours du temps.

7. Procédé selon la revendication 5 ou 6, **caractérisé en ce que** les données de mesure électrique sont des données d'impédance électrique et/ou de conductivité électrique du fluide corporel.

8. Procédé selon l'une des revendications 5 à 7, **caractérisé en ce que** les capacités d'absorption distinctes des capteurs sont réalisées en jouant sur le type de matériau absorbant employé et/ou sur le volume de matériau absorbant employé et/ou sur la densité du matériau absorbant employé.

## Patentansprüche

1. Vorrichtung (1) zur Überwachung des Schwitzens eines Lebewesens, wobei das Schwitzen die Bildung einer Körperflüssigkeit bewirkt, wobei die Vorrichtung einen Träger (10) beinhaltet, der eine aktive Oberfläche (100) aufweist, die dazu bestimmt ist, an die Haut (P) eines Lebewesens angelegt zu werden, wobei die Vorrichtung eine Verarbeitungseinheit (UC) umfasst und auch n Sensoren mit n größer oder gleich 2 umfasst, wobei jeder Sensor (S1-Sn) auf der aktiven Oberfläche angeordnet ist und mit der Verarbeitungseinheit (UC) verbunden ist, um Datenelemente einer elektrischen Messung zu liefern, wobei jeder Sensor ein Absorptionselement (2) umfasst, das in der Lage ist, ein Körperflüssigkeitsvolumen zu absorbieren, wobei die Sensoren mit unterschiedlichen Körperflüssigkeitsabsorptionskapazitäten ausgewählt werden, so dass ein erster Sensor schneller mit Körperflüssigkeit gesättigt wird als ein zweiter Sensor, wobei die Verarbeitungseinheit (UC) dazu ausgelegt ist, eine Ionenkonzentration der Körperflüssigkeit anhand des von dem ersten Sensor (S1) bei Sättigung gelieferten Werts des Datenelements einer elektrischen Messung und des maximalen Körperflüssigkeitsvolumens, welches das Absorptionselement (2) des ersten Sensors (S1) absorbieren kann, zu bestimmen, wobei die Verarbeitungseinheit (UC) dazu ausgelegt ist, die volumenbezogene Füllung des zweiten Sensors (S2) im Laufe der Zeit unter Berücksichtigung der von diesem zweiten Sensor (S2) im Laufe der Zeit gelieferten Datenelemente einer elektrischen Messung und der mithilfe des ersten Sensors (S1) bestimmten Ionenkonzentration zu verfolgen.

2. Vorrichtung nach Anspruch 1, **dadurch gekennzeichnet, dass** die Verarbeitungseinheit (UC) dazu ausgelegt ist, das von dem ersten Sensor (S1) absorbierte Körperflüssigkeitsvolumen unter Berücksichtigung der bestimmten Ionenkonzentration der Körperflüssigkeit und der von dem ersten Sensor (S1) im Laufe der Zeit gelieferten Datenelemente einer elektrischen Messung zu bestimmen.

3. Vorrichtung nach Anspruch 1 oder 2, **dadurch gekennzeichnet, dass** die Datenelemente einer elektrischen Messung Datenelemente einer elektrischen Impedanz und/oder einer elektrischen Leitfähigkeit der Körperflüssigkeit sind.

4. Vorrichtung nach einem der Ansprüche 1 bis 3, **dadurch gekennzeichnet, dass** die unterschiedlichen Absorptionskapazitäten der Sensoren realisiert werden, indem auf die Art des verwendeten absorbierenden Materials und/oder auf das Volumen des verwendeten absorbierenden Materials und/oder auf die Dichte des verwendeten absorbierenden Materials eingewirkt wird.

5. Verfahren zur Überwachung des Schwitzens eines Lebewesens, wobei das Schwitzen die Bildung einer Körperflüssigkeit bewirkt, wobei das Verfahren **dadurch gekennzeichnet ist, dass** es mit einer Vorrichtung (2) zur Überwachung wie in einem der vorhergehenden Ansprüche definiert durchgeführt wird und dass es darin besteht, die Ionenkonzentration der Körperflüssigkeit anhand des von dem ersten Sensor (S1) bei Sättigung gelieferten Werts des Datenelements einer elektrischen Messung und des maximalen Körperflüssigkeitsvolumens, welches das Absorptionselement des ersten Sensors absorbieren kann, zu bestimmen, wobei das Verfahren einen Schritt des Überwachens der volumenbezogenen Füllung des zweiten Sensors (S2) im Laufe der Zeit unter Berücksichtigung der von diesem zweiten Sensor (S2) im Laufe der Zeit gelieferten Datenelemente einer elektrischen Messung und der mithilfe des ersten Sensors bestimmten Ionenkonzentration umfasst.

6. Verfahren nach Anspruch 5, **dadurch gekennzeichnet, dass** es einen Schritt des Bestimmens des von dem ersten Sensor (S1) absorbierten Körperflüssigkeitsvolumens unter Berücksichtigung der bestimmten Ionenkonzentration der Körperflüssigkeit und der von dem ersten Sensor (S1) im Laufe der Zeit gelieferten Datenelemente einer elektrischen Messung umfasst.

7. Verfahren nach Anspruch 5 oder 6, **dadurch gekennzeichnet, dass** die Datenelemente einer elektrischen Messung Datenelemente einer elektrischen Impedanz und/oder einer elektrischen Leitfähigkeit der Körperflüssigkeit sind.

8. Verfahren nach einem der Ansprüche 5 bis 7, **dadurch gekennzeichnet, dass** die unterschiedlichen Absorptionskapazitäten der Sensoren realisiert werden, indem auf die Art des verwendeten absorbierenden Materials und/oder auf das Volumen des verwendeten absorbierenden Materials und/oder auf die Dichte des verwendeten absorbierenden Materials eingewirkt wird.

## Claims

1. Device (1) for monitoring the perspiration of a living being, said perspiration causing the creation of a bodily fluid, said device comprising a support (10) having an active surface (100) intended to be applied against the skin (P) of a living being, said device having a processing unit (UC) and also having n sensors, with n greater than or equal to 2, each sensor (S1-Sn) being arranged on said active surface and connected to said processing unit (UC) for supplying electrical-measurement data, each sensor having an absorption element (2) capable of absorbing a volume of bodily fluid, the sensors being chosen with distinct bodily-fluid absorption capacities such that a first sensor will become saturated with bodily fluid more quickly than a second sensor, the processing unit (UC) being configured to determine an ion concentration of the bodily fluid, from the value for the electrical-measurement datum supplied by the saturated first sensor (S1) and from the maximum volume of bodily fluid that the absorption element (2) of the first sensor (S1) can absorb, the processing unit (UC) being configured to track the volumetric filling of the second sensor (S2) over time while taking into account electrical-measurement data supplied by this second sensor (S2) over time and the ion concentration determined by means of the first sensor (S1).

2. Device according to Claim 1, **characterized in that** the processing unit (UC) is configured to determine the volume of bodily fluid absorbed by the first sensor (S1) while taking into account the determined ion concentration of the bodily fluid and electrical-measurement data supplied by the first sensor (S1) over time.

3. Device according to Claim 1 or 2, **characterized in that** the electrical-measurement data are data about the electrical impedance and/or the electrical conductivity of the bodily fluid.

4. Device according to one of Claims 1 to 3, **characterized in that** the distinct absorption capacities of the sensors are realized by varying the type of absorbent material used and/or the volume of absorbent material used and/or the density of the absorbent material used.

5. Method for monitoring the perspiration of a living being, said perspiration causing the creation of a bodily fluid, said method being **characterized in that** it is implemented by means of a monitoring device (2) as defined in one of the preceding claims, and **in that** it consists in determining the ion concentration of the bodily fluid, from the value for the electrical-measurement datum supplied by the saturated first sensor (S1) and from the maximum volume of bodily fluid that the absorption element of the first sensor can absorb, said method having a step of monitoring the volumetric filling of the second sensor (S2) over time while taking into account electrical-measurement data supplied by this second sensor (S2) over time and the ion concentration determined by means of the first sensor.

6. Method according to Claim 5, **characterized in that** it has a step of determining the volume of bodily fluid absorbed by the first sensor (S1) while taking into account the determined ion concentration of the bodily fluid and electrical-measurement data supplied by the first sensor (S1) over time.

7. Method according to Claim 5 or 6, **characterized in that** the electrical-measurement data are data about the electrical impedance and/or the electrical conductivity of the bodily fluid.

8. Method according to one of Claims 5 to 7, **characterized in that** the distinct absorption capacities of the sensors are realized by varying the type of absorbent material used and/or the volume of absorbent material used and/or the density of the absorbent material used.
